Europäisches Patentamt

European Patent Office

Office européen des brevets

(19)

(11) Veröffentlichungsnummer: **0 034 763**
**B1**

(12) **EUROPÄISCHE PATENTSCHRIFT**

(45) Veröffentlichungstag der Patentschrift:
06.07.83

(21) Anmeldenummer: **81100973.7**

(22) Anmeldetag: **12.02.81**

(51) Int. Cl.³: **C 07 C 120/00,** C 07 C 121/66,
C 07 C 121/68, C 07 C 121/75,
C 07 D 333/24

(54) **Verfahren zur Gewinnung reiner Nitrile.**

(30) Priorität: **21.02.80 DE 3006424**

(43) Veröffentlichungstag der Anmeldung:
**02.09.81 Patentblatt 81/35**

(45) Bekanntmachung des Hinweises auf die Patenterteilung:
**06.07.83 Patentblatt 83/27**

(84) Benannte Vertragsstaaten:
**AT BE DE FR GB IT NL SE**

(56) Entgegenhaltungen:
**DE-A-2 449 013**
**US-A-2 553 404**
**Chemical Abstracts Band 83, Nr. 15, 13. Oktober 1975 Columbus, Ohio, USA R. A. GRIMM et al. »Phenylacetonitriles and phenylmalononitriles from toluenes and cyanogen chloride« Seite 477, Spalte 1, Abstract Nr. 131296n**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Greifenhagenstrasse 25, D-6450 Hanau 9 (DE)**
Erfinder: **Schalke, Peter M., Dr., Südring 74, D-6458 Rodenbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Gewinnung reiner Nitrile

Arylacetonitrile werden durch Umsetzung von Chlorcyan und aromatischen oder heteroaromatischen Methylen- bzw. Methylverbindungen, die aktivierten Wasserstoff enthalten, bei Temperaturen von 500° C bis herauf zu 1200° C hergestellt, siehe Grimm/Menting, Ind. Eng. Chem. Prod. Res. Div. Vol. 14 (1975), No. 3, Seite 158 – 161 sowie US-PS 2 553 404 und 2 606 917.

Weitere Verbindungen, die durch Umsetzung von Chlorcyan und entsprechenden Methyl- oder Methylenverbindungen hergestellt werden können, wurden in der DE-OS 2 854 210 vorgeschlagen.

Bei der Herstellung der Nitrile nach diesen Verfahren besteht die Schwierigkeit, aus den heißen Reaktorgasen das Produkt und das im Überschuß eingesetzte bzw. nicht umgesetzte Ausgangsmaterial auf einfache Weise und unter Vermeidung von Verlusten zu isolieren, wobei eine rasche Abtrennung oder Neutralisation der entstandenen Salzsäure erreicht werden muß.

Im Aufsatz von »Grimm und Menting« (loc.cit.), der sich neben der Herstellung von Phenylacetonitril auch mit der von Phenylmalonsäuredinitril befaßt, werden für die Isolierung von Phenylacetonitril aus dem Reaktionsgasgemisch zwei verschiedene Isolierungsmethoden beschrieben.

Bei einfacher Kühlung mittels eines Rückflußkühlers — siehe »Grimm-Menting« loc. cit. — gelang das Niederschlagen des bei der Kondensation entstehenden feinen Gasnebels nur sehr unvollkommen. Da bei dieser Arbeitsweise auf eine Neutralisation der Salzsäure verzichtet wird, kann diese wiederum zersetzend auf Produkt und Ausgangssubstanz einwirken.

Dabei führte die direkte Destillation des entstandenen Reaktionsgasgemisches bei Phenylacetonitril zu einem nicht farbstabilen Produkt. Auch die Umwandlung dieses Nitrils in ein farbstabiles Acetat war nur mit technischen Schwierigkeiten durchführbar.

Ebenso traten beim Auffangen des Reaktionsgases in einem Methanolbad Schwierigkeiten auf, da ein Teil der Nitrile in die entsprechenden Ester umgewandelt wurde.

In der US-PS 2 553 404 und 2 606 917 wird auf prinzipiell gleiche Weise kondensiert. In der US-PS 2 606 917 entsteht durch bereits in den Reaktor miteingespeisten Dampf beim Kondensieren eine konzentrierte Salzsäurelösung, die das Produkt angreift und es teilweise verseift.

Aus diesem Grund sind die in diesen Patenten angegebenen Ausbeuten deutlich niedriger als bei den anderen bekannten Verfahren.

Wasser oder wasserhaltiges Kühlmittel werden auch als direkte Kondensationsmittel eingesetzt, s. DE-OS 2 854 210. Die dadurch gebildete wäßrige Salzsäure kann jedoch stark korrodierend auf die Apparatur und auch verseifend auf das gebildete Produkt wirken. Aus diesem Grund wurde das Reaktionsgasgemisch dann während der Abkühlung gleichzeitig neutralisiert nach diesem Vorschlag.

In der DE-OS 2 854 210 wird empfohlen, das den Reaktor verlassende Gasgemisch direkt auf eine Temperatur unter 100° C abzukühlen oder abzuschrecken und damit das Gas zur Kondensation zu bringen. Günstig soll es hiernach auch sein, in die Gase zwecks schneller Kühlung ein Kühlmittel, vorzugsweise Wasser, einzuspritzen. Das Wasser kann dabei zur Neutralisation der bei der Reaktion entstandenen Salzsäure basisch wirkende Stoffe enthalten, wie z. B. Alkalihydroxide, Alkalicarbonate oder -bicarbonate. Schwierigkeiten treten aber hierbei durch das entstehende Abwasser auf, das noch hochtoxische Stoffe, wie z. B. in Wasser gelöste Ausgangssubstanz, Produkt, nicht umgesetztes Chlorcyan, Neben- und Zersetzungsprodukte der Reaktion, enthält und schwierig zu beseitigen ist.

Da die Kühlmittel aus Gründen der besseren Verteilung in dem Reaktionsgas und damit zu dessen schnellerer Abkühlung im allgemeinen in das Reaktionsgas eingespritzt werden, kommt noch zusätzlich die Gefahr einer Düsenverstopfung durch die in dem kondensierten Produkt anwesenden Rußteilchen oder festen Neutralisationsprodukte hinzu. Die — bei der Verwendung von Wasser oder wasserhaltigen Kühlmitteln — an der Einspritzstelle bei hoher Temperatur entstehende chloridhaltige Lösung kann auch zu Korrosionsproblemen führen. Diese sind mit an dieser Stelle notwendigen metallischen Werkstoffen nicht oder nur schwer zu bewältigen.

Sehr nachteilig bei der technischen Gewinnung der Nitrile ist ferner die Tatsache, daß die Nitrile, die empfindlich gegenüber Salzsäure sind, bei der bisher üblichen destillativen Aufarbeitung des rohen Reaktionsgemisches über längere Zeit neben der gebildeten Salzsäure bzw. dem Chlorwasserstoff vorlagen.

Erst nach Abdestillieren des neben Chlorcyan eingesetzten Reaktionspartners, der in zwei- bis dreifachem Überschuß eingesetzt wird, wurde mit dem Abdestillieren des gebildeten Nitrils begonnen. Inzwischen war aber das Nitril selbst schon erheblich angegriffen worden. Es kommt noch hinzu, daß der jeweilige Reaktionspartner von Chlorcyan generell thermisch stabiler ist als das aus ihm gebildete Nitril.

Es leiden also bei diesen Arbeitsweisen sowohl Ausbeute wie Reinheit des isolierten Produktes und auch des rückgewonnenen Ausgangsmaterials; außerdem entsteht ein höherer Anteil an Destillationssumpf.

Die bekannten Kondensierungsmethoden führen also zu Substanzverlusten oder zu technischen Schwierigkeiten bei der Gewinnung des betreffenden Nitrils aus dem Reaktionsgasgemisch.

Zweck der Anmeldung ist daher die Angabe eines technisch einfachen Verfahrens zur Kondensation der Reaktorgase und damit Erhöhung der Ausbeute an Nitril.

Es wurde nun gefunden, daß man die Nitrile, die bei der Umsetzung von Chlorcyan mit aromatischen oder heteroaromatischen Methylenverbindungen bzw. Methylverbindungen, die aktivierten Wasserstoff enthalten, mit einer verbesserten Isolierungsmethode und in hoher Ausbeute aus dem Reaktionsgas gewinnen kann, wenn man das nach der Umsetzung bei $500-1200°C$ anfallende Reaktionsgasgemisch in den aufeinanderfolgenden Stufen so kondensiert, daß man das Reaktionsgas — gegebenenfalls im Gleichstrom mit einem Inertgas — in eine erste Kondensationsstufe, deren Temperaturgefälle zwischen dem Siedepunkt der eingesetzten Methylen- bzw. Methylverbindung und dem Siedepunkt des jeweiligen in der Reaktion gebildeten Nitrils liegt, einführt, fraktioniert kondensiert, das kondensierte rohe Nitril als Sumpfprodukt abzieht, den nicht kondensierten Anteil, der in der Hauptsache aus der nicht umgesetzten Methyl- oder Methylenverbindung und Chlorcyan, sowie aus dem während der Reaktion gebildeten Chlorwasserstoff und den Nebenprodukten besteht, in eine zweite Kondensationsstufe führt, deren Temperaturgefälle zwischen Zimmertemperatur und dem Siedepunkt der Methyl- oder Methylenverbindung liegt, kondensiert, das dabei anfallende zweite Kondensat aus restlicher Methyl- oder Methylenverbindung abzieht und bevorzugt das hierbei anfallende Abgas aus Chlorwasserstoff und gegebenenfalls Inertgas in eine dritte Kondensationsstufe einbringt, in der man den Chlorwasserstoff und gegebenenfalls das Inertgas bei Temperaturen von $+10°C$ bis $-100°C$, bevorzugt von $-10$ bis $-70°C$, von geringen Resten an organischen Verunreinigungen trennt, worauf man das anfallende rohe Nitril in bekannter Weise destilliert und gegebenenfalls in bekannter Weise weiter reinigt; das in der zweiten Kondensationsstufe anfallende Kondensat bevorzugt in die Reaktionsstufe zur Herstellung von Nitril zurückführt und bevorzugt den Chlorwasserstoff aus dem die dritte Kondensationsstufe verlassende Gas durch Auswaschen mit Wasser gewinnt.

Chlorcyan und sein jeweiliger Reaktionspartner werden — wie in den bekannten Verfahren — in Form ihrer Gase eingesetzt, ebenso in den bekannten Molverhältnissen, z. B. 1 : 1 bis 1 : 6. Wenn Inertgase während der Fraktionierung verwendet werden sollen, so kommen hierfür besonders Stickstoff und Kohlendioxid in Frage.

Als Reaktionspartner für Chlorcyan werden aromatische oder heteroaromatische Methylen- bzw. Methylverbindungen, die aktivierten Wasserstoff enthalten und mit Salzsäure kein Hydrochlorid bilden, eingesetzt.

Für das erfindungsgemäße Verfahren sind also einmal geeignet am Kern substituierte Toluolderivate. Als Substituenten kommen in Frage: Wasserstoff, Halogene wie Fluor und/oder Chlor, weitere Methylgruppen, Hydroxygruppen oder Nitrilgruppen.

Der Substituent kann wahlweise in o-, m- oder p-Stellung zu der Methylgruppe des Toluols bei dessen entsprechenden Derivaten stehen; die Hydroxygruppe allerdings nur in p-Stellung.

Gemeinsam können als Substituenten beim Toluol auftreten Fluor und Chlor oder zwei Methylgruppen in 3- und 5-Stellung zu der schon vorhandenen Methylgruppe.

Bei Wasserstoff als Substituenten handelt es sich natürlich um Toluol selbst.

Ferner erwiesen sich als geeignet kernsubstituierte Methylthiophene. In Frage kommen besonders 2-Methylthiophen; 3-Methylthiophen; 2,5-Di-methylthiophen; 3,4-Dimethylthiophen.

Eine weitere Verbindung, die sich im erfindungsgemäßen Verfahren einsetzen läßt, ist Diphenylmethan.

Bevorzugt sind: Toluol, o-Xylol, m-Xylol, p-Xylol, o-Fluortoluol, m-Fluortoluol, p-Fluortoluol; p-Kresol; 2-Methylthiophen; 3-Methylthiophen und Diphenylmethan. Von diesen genannten sind besonders bevorzugt Toluol, 2-Methylthiophen und 3-Methylthiophen, P-Cyanotoluol ist auch verwendbar.

Bei den Produkten, die bei dem erfindungsgemäßen Verfahren anfallen, handelt es sich um die entsprechend dem Ausgangsmaterial substituierten Phenylacetonitrile oder Thiophenacetonitrile bzw. um Diphenylacetonitril.

Für die erste und zweite Kondensationsstufe werden bekannte Fraktionierkolonnen, die verschiedenartige Böden oder Füllungen enthalten, verwendet, bevorzugt Sambay- oder Fallfilmverdampfer. Bevorzugt wird das Reaktionsgemisch vorgekühlt in die 1. Stufe eingeleitet. Zur Einstellung der gewünschten Temperatur wird z. B. eine thermostatisierte Flüssigkeit im Doppelmantel der Kolonnen oder Fallfilmverdampfer verwendet, die als unterste Temperatur in der ersten Kondensationsstufe 80°C nicht unterschreiten und 200°C nicht überschreiten soll.

Entsprechendes gilt für die zweite und dritte Kondensationsstufe, d. h., in der zweiten Stufe soll die Temperatur der Flüssigkeit 20°C nicht unterschreiten und 82°C nicht überschreiten; in der dritten Stufe $-100°C$ nicht unterschreiten und $+5°C$ nicht überschreiten.

Als thermostatisierte Flüssigkeit kann in der ersten Kondensationsstufe auch Dampf für Heiztemperaturen über 100°C verwendet werden. Im allgemeinen werden für dieses Temperaturgebiet die hierfür bekannten Öle eingesetzt.

Die optimalen Temperaturen für die Betreibung der Fraktionierkolonnen in den einzelnen Kondensationsstufen werden mit Hilfe analytischer Messungen durch einen Vorversuch festgelegt, da sie bekanntlich von üblichen Kolonnenparametern wie Höhe, Durchmesser, Art der Füllkörper oder Böden (wenn vorhanden), Einspeisemengen und Strömungsgeschwindigkeiten der zu kondensierenden Gase abhängen.

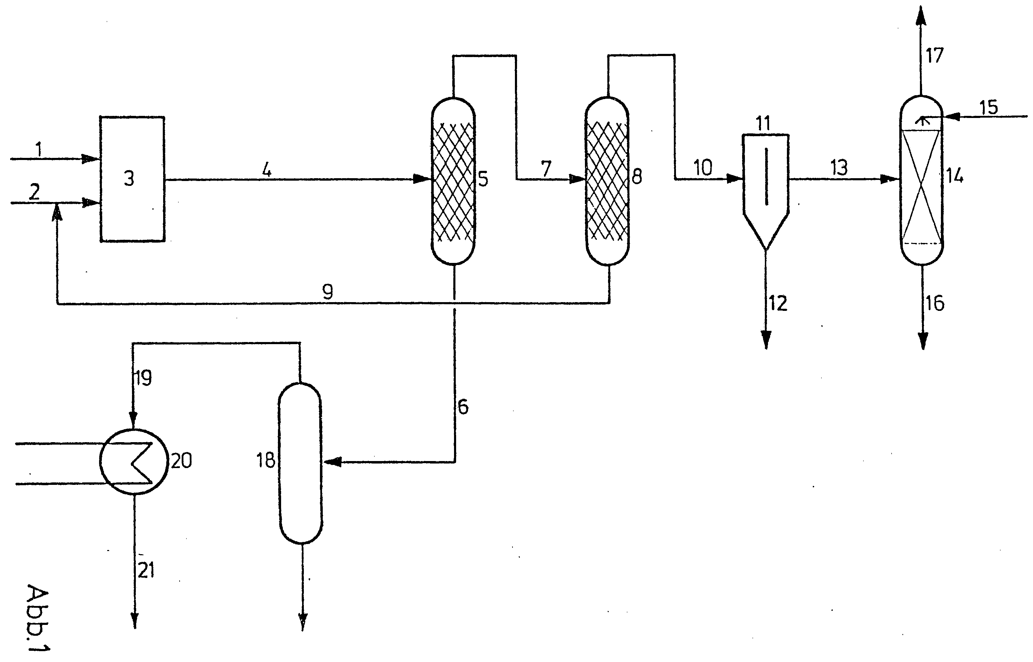

Die direkte Fraktionierung des Reaktionsgases wird anhand der schematischen Abbildung 1 erläutert.

Über Leitung 1 wird Chlorcyan, über Leitung 2 der Reaktionspartner in den Reaktor 3 eingeführt und dort umgesetzt.

Aus dem Reaktor 3 tritt das nitrilhaltige Gas über die gekühlte Leitung 4 in die Fraktionierkolonne 5 ein, wo die Trennung in rohes Nitril, das über Leitung 6 abgenommen wird, und gasförmigen Reaktionspartner erfolgt, der zusammen mit ebenfalls gasförmigem Chlorwasserstoff, nicht umgesetztem Chlorcyan und organischen Nebenprodukten am Kopf der Kolonne 5 über Leitung 7 abgenommen und in die Fraktionierkolonne 8 geleitet wird.

Hier werden der nicht umgesetzte Reaktionspartner und Chlorcyan möglichst vollständig kondensiert und entweder als solche entnommen (nicht gezeigt) oder bevorzugt über Leitung 9 in den Reaktor 3 zur Herstellung von Nitril zurückgeführt.

Das die Kolonne 8 verlassende Restgas besteht im wesentlichen aus Chlorwasserstoff und gegebenenfalls Inertgas, sowie leichter siedenden Nebenprodukten, wie z. B. Addukten aus Chlorcyan und Chlorwasserstoff mit unbekannter Struktur.

In Abbildung 1 wird dieses Restgas über Leitung 10 in den Abscheider 11 geleitet und dort von den organischen Verunreinigungen befreit, die über Leitung 12 flüssig abgenommen und — falls gewünscht — gesondert aufgearbeitet werden können. (Letzteres nicht gezeigt.)

Der jetzt von Verunreinigungen freie gasförmige Chlorwasserstoff gelangt über Leitung 13 in eine Waschkolonne 14, in die über Leitung 15 Wasser eingeführt wird. Die wäßrige salzsaure Lösung verläßt über Leitung 16 das System. Je nach Menge des zugeführten Wassers können konzentrierte oder weniger konzentrierte salzsaure Lösungen gewonnen werden. Auch die Verwendung anderer Waschmittel wie alkalischer Lösungen, z. B. von verdünnten Alkalilaugen oder -Carbonatlösungen, ist an sich möglich; sie führen aber nicht zu verwertbaren Produkten, wie bei der Verwendung von Wasser.

Die mögliche Verwendung von Inertgas, das über Leitung 17 das System verläßt, für die Aufarbeitung des Reaktionsgases wurde nicht gezeigt.

Das die Fraktionierkolonne 5 über Leitung 6 verlassende rohe Nitril tritt in die Fraktionierkolonne 18 ein, aus der das reine Nitril in Dampfform über Leitung 19 abgenommen und in dem Kühler 20 kondensiert wird. Das Produkt wird über Leitung 21 abgenommen.

Für die erste und zweite Kondensationsstufe 5 und 8 werden bekannte Fraktionierkolonnen, die verschiedenartige Böden oder Füllungen enthalten, verwendet, bevorzugt Sambay- oder Fallfilmverdampfer. Sie werden unter Apparatedruck betrieben, bevorzugt Normaldruck. Die dritte Kondensationsstufe 11 besteht im allgemeinen aus einem Wärmetauscher, der mit Kühlsole betrieben wird. Diesem Wärmetauscher kann ein Aktivkohlefilter zur besseren Adsorption der Nebenprodukte vor- oder nachgeschaltet sein. Anstelle von Aktivkohle können auch andere Adsorptionsmittel verwendet werden.

Das Verfahren gestattet die direkte Isolierung des in der Reaktion gebildeten Nitrils aus dem Reaktionsgas unter Erhöhung der Ausbeute, ferner die direkte Isolierung der im Überschuß eingesetzten Ausgangssubstanz, die dann sofort in einen Kreisprozeß, gegebenenfalls mit darin enthaltenen Resten an in der Reaktion nicht umgesetztem Chlorcyan, in den Reaktor rezykliert werden kann, sowie die Gewinnung von salzsäurehaltigen Lösungen. Werden die salzsäurehaltigen Lösungen wieder verwendet, so fällt kein Abwasser an.

### Beispiel 1

#### Phenylacetonitril aus Toluol

Über Leitung 1 werden pro Stunde 86,1 g (1,4 Mol) gasförmiges Chlorcyan und über Leitung 2 561 g (6,1 Mol) gasförmiges Toluol sowie 1 bis 2 l gasförmiger Stickstoff in den auf einem Meter mit einer Durchschnittstemperatur von 680° C beheizten Reaktor 3 von einem Durchmesser von 55 mm eingespeist. Aus dem Reaktor 3 tritt das nitrilhaltige Gas über die gekühlte Leitung 4 in die Kolonne 5 ein. Die Kolonne 5 besteht aus einem »Sambay-Verdampfer« mit einer Innenoberfläche von 0,1 m² und einer aufgesetzten Füllkörperkolonne von 20 cm Länge. Der Doppelmantel der Kolonne 5 ist mit heißem Öl durchflossen. Am Fuß der Kolonne werden pro Stunde 2 bis 3 l gasförmiger Stickstoff eingespeist. Die Temperatur des Öls im Doppelmantel der Kolonne 5 ist so geregelt, daß am Fuß der Kolonne 5 das in der Kolonne kondensierende Phenylacetonitril mit einer Temperatur von ca. 140° C über Leitung 6 entnommen werden kann, während am Kopf der Kolonne 5 das Abgas mit ca. 120° C über Leitung 7 in die Fraktionierkolonne 8 geleitet wird. Die Kolonne 8 hat eine Länge von 50 cm, einen Durchmesser von 2 cm und ist mit Raschigringen gefüllt. Am Fuß der Kolonne werden pro Stunde ca. 2−3 l gasförmiger Stickstoff eingeleitet. Die Außenkühlung über Doppelmantel ist so geregelt, daß am Fuß der Kolonne eine Sumpftemperatur von 110° C und am Kolonnenkopf eine Temperatur von 20−40° C herrschen. Das in der Kolonne 8 kondensierte Toluol mit darin enthaltenen Resten an Chlorcyan wird über Leitung 9 entnommen, zur Leitung 2 rückgeführt und vermischt mit frischem Toluol zur Ergänzung der verbrauchten Substanz in den Reaktor 3 geleitet.

Das die Kolonne 8 verlassende Restgas wird über Leitung 10 in einen bei −30° C gehaltenen Abscheider 11, bestehend aus einer Kühlfalle, über Leitung 13 durch ein Aktivkohlefilter in eine Waschkolonne 14 geführt, in der ein Auswaschen des Restgases mit Wasser erfolgt. In der Kolonne 5 fallen pro Stunde 146,9 g Rohprodukt an, aus dem durch Destillation in der Kolonne 18 bei 70° C/3 mm 1,8 g o-Toluinitril abgetrennt werden. Anschließend destilliert bei 85° C/3 mm das reine Phenylacetonitril, von dem 142,4 g mit einer Reinheit von 99% erhalten werden. Das entspricht einer Ausbeute von 86%, bezogen auf eingesetztes Chlorcyan.

In der Kolonne 8 fallen pro Stunde 429,4 g Lösung an. Für das rückgewonnene Toluol wird eine Reinheit von etwa 99% ermittelt. Es sind etwa 0,4% Chlorcyan nachweisbar. Damit errechnet sich eine Ausbeute von 84%, bezogen auf Toluol.

Die Titration der wäßrigen Lösung des Gaswäschers mit Natronlauge gegen Methylorange als Indikator ergibt einen HCl-Gehalt, der 98,5% der aus dem Chlorcyan entstandenen Salzsäure entspricht.

In dem Vergleichsbeispiel wird die methanolfreie Kondensationsmethode von »Grimm und Menting« nachgearbeitet.

### Vergleichsbeispiel

Über Leitung 1 werden pro Stunde 86 g (1,4 Mol) gasförmiges Chlorcyan und über Leitung 2 561 g (6,1 Mol) gasförmiges Toluol und 2 l gasförmiger Stickstoff in den gleichen Reaktor 3 bei 680° C eingespeist. Aus dem Reaktor 3 wird das Produktgas in einen durch ein Wasserbad gekühlten Dreihalskolben mit aufgesetztem Rückflußkühler eingeleitet. Das den Rückflußkühler verlassende Abgas wird über den Abscheider 11 in die Waschkolonne 14 geleitet.

Durch Kondensation des Reaktionsgases werden pro Stunde im Kolben und im Abscheider 584,4 g Lösung erhalten, die durch fraktionierte Destillation aufgearbeitet werden.

Bei einer Siedetemperatur von 110° C werden aus einem einstündigen Versuch 397,5 g Toluol zurückerhalten. Anschließend werden durch Destillation bei 70° C/3 mm 5,2 g o-Toluinitril gewonnen. Danach destillieren 131 g Phenylacetonitril bei 85° C/3 mm.

Als Destillationssumpf verbleiben 44,2 g. Damit errechnet sich eine Ausbeute von 79,9%, bezogen auf Chlorcyan und 62,9%, bezogen auf Toluol.

Bei der Destillation wird aus der Lösung eine nicht unbeträchtliche Menge an Salzsäure sowie etwas Chlorcyan ausgetrieben. Die Titration des Inhalts der Waschkolonne 14 mit Natronlauge gegen Methylorange als Indikator ergibt einen HCl-Gehalt, der nur 62,3% der aus dem Chlorcyan entstandenen Salzsäure entspricht.

### Beispiel 2

Thiophen-3-acetonitril aus 3-Methylthiophen

Es wird die gleiche Apparatur verwendet, wie sie im Beispiel 1 beschrieben ist.

In den auf 710° C beheizten Reaktor werden pro Stunde 49,2 g (0,8 Mol) gasförmiges Chlorcyan, 314,1 g (3,2 Mol) gasförmiges 3-Methylthiophen und 2 l Stickstoff eingespeist. Am Fuße der Kolonne 5 wird das Rohprodukt mit ca. 135° C entnommen und in die Fraktionierkolonne 18 aufdestilliert. In der Kolonne 5 fallen pro Stunde 88,1 g Rohprodukt an, aus dem durch Destillation in der Kolonne 18 bei 115–120° C/12 mm 83,4 g Thiophen-3-acetonitril mit einer Reinheit von 98% erhalten werden. Das entspricht einer Ausbeute von 82,9%, bezogen auf eingesetztes Chlorcyan.

Das Abgas der Kolonne 5 wird mit etwa 120° C in die Kolonne 8 geleitet. Das in der Kolonne 8 kondensierte 3-Methylthiophen wird mit einer Sumpftemperatur von 110° C entnommen, mit darin enthaltenen Resten an Chlorcyan und frischem 3-Methylthiophen zur Ergänzung an verbrauchter Substanz in den Reaktor rückgeführt. In der Kolonne 8 fallen pro Stunde 236,5 g Lösung an. Sie besteht zu 98,3% aus 3-Methylthiophen und enthält ca. 0,6% Chlorcyan. Damit errechnet sich eine Ausbeute von 79,8%, bezogen auf 3-Methylthiophen.

Das die Kolonne 8 verlassende Restgas wird über Leitung 10 in einen bei −30° C gehaltenen Abscheider in eine Waschkolonne 14 geführt. Die Titration der wäßrigen Lösung dieses Gaswäschers mit Natronlauge gegen Methylorange als Indikator ergibt einen HCl-Gehalt, der 96,2% der aus dem Chlorcyan entstandenen Salzsäure entspricht.

### Vergleichsbeispiel

Der Reaktor wird in der gleichen Weise betrieben wie im Beispiel 2, jedoch mit der Quenchapparatur des Vergleichsbeispiels 1 (Phenylacetonitril). Es werden pro Stunde 324,6 g Lösung erhalten, die durch fraktionierte Destillation aufgearbeitet werden.

Bei einer Siedetemperatur von 114–116° C werden aus einem einstündigen Versuch 221,5 g 3-Methylthiophen zurückerhalten. Anschließend werden durch Vakuumdestillation bei 115–120° C/12 mm 77,4 g Thiophen-3-acetonitril mit einer Reinheit von 98% gewonnen. Als Destillationssumpf verbleiben 25,1 g. Damit errechnet sich eine Ausbeute von 77%, bezogen auf Chlorcyan und 65,3%, bezogen auf 3-Methylthiophen. Die Titration des Inhalts der Waschkolonne ergibt einen HCl-Gehalt, der nur 47,3% der aus dem Chlorcyan entstandenen Salzsäure entspricht.

### Beispiel 3

Thiophen-2-acetonitril aus 2-Methylthiophen

Es wird die gleiche Apparatur verwendet, wie sie im Beispiel 1 beschrieben ist.

In den auf 690° C beheizten Reaktor werden pro Stunden 86 g (1,4 Mol) gasförmiges Chlorcyan, 549 g (5,6 Mol) gasförmiges 2-Methylthiophen, sowie 1 – 2 l gasförmiger Stickstoff eingespeist.

In der Kolonne 5 fallen pro Stunde 155,9 g Rohprodukt mit einer Temperatur von 135° C an, aus denen nach Destillation in der Kolonne 18 bei 113 – 115° C/12 mm 148 g Thiphen-2-acetonitril mit einer Reinheit von 98% gewonnen werden. Das entspricht einer Ausbeute von 84,2%, bezogen auf eingesetztes Chlorcyan.

Das bei 115° C in die Kolonne 8 übergehende Gas wird hier kondensiert, wobei 397 g Lösung pro Stunde mit einer Temperatur von 110° C erhalten werden. Die Lösung besteht zu 99% aus 2-Methylthiophen und enthält noch 0,5% Chlorcyan. Damit errechnet sich eine Ausbeute von 75%, bezogen auf 2-Methylthiophen. Die Lösung wird sofort in den Reaktor rückgeführt.

Die Titration des Inhalts des Gaswäschers ergibt einen HCl-Gehalt, der 95,3% der aus dem Chlorcyan entstandenen Salzsäure entspricht.

### Vergleichsbeispiel

Bei Durchführung des Vergleichsbeispiels analog den Vergleichsbeispielen 1 und 2 können 405,7 g 2-Methylthiophen mit einer Reinheit von 99%, 118,1 g Thiophen-2-acetonitril mit einer Reinheit von 98% und 42 g Destillationssumpf erhalten werden. Die Titration ergibt einen HCl-Gehalt von 53,2%. Es errechnen sich Ausbeuten von 68,6%, bezogen auf Chlorcyan und 65,6%, bezogen auf 2-Methylthiophen.

### Beispiel 4

### 4-Methylphenylacetonitril aus 1,4-Dimethylbenzol

Über Leitung 1 werden pro Stunde 53,4 g gasförmiges Chlorcyan und über Leitung 2 386 g gasförmiges 1,4-Dimethylbenzol sowie 1 – 2 l gasförmiger Stickstoff in einen auf 110 cm mit einer Durchschnittstemperatur von 720° C beheizten Reaktor 3 von einem Durchmesser von 60 mm eingespeist. Aus dem Reaktor 3 tritt das produkthaltige Gas über die gekühlte Leitung 4 in die Kolonne 5 ein. Das in der Kolonne 5 kondensierende 4-Methylphenylacetonitril wird mit einer Temperatur von ca. 170° C über Leitung 6 entnommen und der Kolonne 18 zur Vakuumdestillation zugeleitet.

Am Kopf der Kolonne 5 wird das Abgas mit etwa 150° C über Leitung 7 in die Kolonne 8 geleitet. Das in der Kolonne 8 kondensierende 1,4-Dimethylbenzol mit darin enthaltenen Resten an Chlorcyan wird bei einer Sumpftemperatur von 110° C entnommen und versetzt mit frischer Substanz in den Reaktor rückgeführt. Das die Kolonne 8 verlassende Restgas wird über Abscheider 11 zur Waschkolonne 14 geführt.

In der Kolonne 5 fallen pro Stunde 96,3 g Rohprodukt an, aus dem durch Destillation in der Kolonne 18 bei 92 – 95° C/3 mm 90,7 g 4-Methylphenylacetonitril mit einer Reinheit von 98% erhalten werden. Das entspricht einer Ausbeute von 78%, bezogen auf eingesetztes Chlorcyan.

In der Kolonne 8 fallen pro Stunde 290,4 g 1,4-Dimethylbenzol mit einer Reinheit von 99% und 0,3% Chlorcyan-Gehalt an. Damit errechnet sich eine Ausbeute von 73%, bezogen auf 1,4-Dimethylbenzol. Die Titation der wäßrigen Lösung des Gaswäschers ergibt einen HCl-Gehalt von 97,3%.

Im Vergleichsbeispiel werden eine Ausbeute von 77%, bezogen auf Chlorcyan und 66%, bezogen auf 1,4-Dimethylbenzol, erhalten.

### Beispiel 5

### 4-Hydroxybenzylcyanid aus p-Kresol

Über Leitung 1 werden pro Stunde 46,1 g (0,75 Mol) gasförmiges Chlorcyan und über Leitung 2 313,6 g (2,9 Mol) gasförmiges p-Kresol sowie 1 – 2 l gasförmiger Stickstoff in den auf einem Meter mit einer Durchschnittstemperatur von 720° C beheizten Reaktor von einem Durchmesser von 55 mm eingespeist. Aus dem Reaktor 3 tritt das nitrilhaltige Gas über die gekühlte Leitung 4 in die Kolonne 5 ein. Die Kolonne 5 besteht aus einem »Sambay-Verdampfer« mit einer Innenoberfläche von 0,1 m² und einer aufgesetzten Füllkörperkolonne von 20 cm Länge. Am Fuß der Kolonne werden pro Stunde 2 – 3 l gasförmiger Stickstoff eingespeist, die Temperatur des Öls im Doppelmantel der Kolonne 5 ist so geregelt, daß am Fuß der Kolonne das in der Kolonne kondensierende Rohprodukt mit einer Temperatur von ca. 180° C über Leitung 6 entnommen werden kann, während am Kopf der Kolonne 5 das Abgas mit etwa 160° C über Leitung 7 in die Fraktionierkolonne 8 geleitet wird. Die Kolonne 8 hat eine Länge von 50 cm, einen Durchmesser von 2 cm und ist mit Raschigringen gefüllt. Am Fuß der Kolonne 8 werden pro Stunde etwa 2 – 3 l gasförmiger Stickstoff eingeleitet. Die Auskühlung über Doppelmantel ist so geregelt, daß am Fuß der Kolonne eine Sumpftemperatur bis zu 120° C und am Kolonnenkopf eine Temperatur von 20 – 40° C herrschen. Das in der Kolonne 8 kondensierte p-Kresol enthält 5 – 6% Phenol. Erst nach destillativem Abtrennen des Phenols kann das rückgewonnene p-Kresol vermischt mit frischem p-Kresol zur Ergänzung verbrauchter Substanz in den Reaktor 3 rückgeführt werden. Das die Kolonne 8 verlassende Restgas wird über Leitung 10 in den bei −30° gehaltenen Abscheider 11 sowie dem Aktivkohlefilter (nicht gezeigt) und darauf in die Waschkolonne 14 geführt, in der das Auswaschen des Restgases mit Wasser erfolgt. In der Kolonne 5 fallen pro Stunde 91,6 g Rohprodukt an, aus dem durch Destillation in der Kolonne 18 bei 160° C/1 Torr 54,9 g p-Hydroxyphenylacetonitril erhalten werden. Das entspricht einer Ausbeute von 55%, bezogen auf eingesetztes Chlorcyan.

In der Kolonne 8 fallen pro Stunde 233,8 g Lösung an. Diese Lösung besteht zu 94,6% aus p-Kresol und zu 5,4% aus Phenol. Damit errechnet sich eine Ausbeute von 48,2%, bezogen auf p-Kresol. Die Titration der wäßrigen Lösung des Gaswäschers mit Natronlauge gegen Methylorange als Indikator ergibt einen HCl-Gehalt, der 91,2% der aus dem Chlorcyan entstandenen Salzsäure entspricht.

**Patentanspruch**

Verfahren zur Gewinnung von Nitrilen, die durch Umsetzung von Chlorcyan mit aromatischen oder heteroaromatischen Methylenverbindungen bzw. Methylverbindungen, die aktivierten Wasserstoff enthalten, hergestellt werden und Aufarbeitung des Reaktionsgemisches, dadurch gekennzeichnet, daß man das nach der Umsetzung bei 500—1200°C anfallende Reaktionsgasgemisch derartig in den aufeinanderfolgenden Temperaturstufen kondensiert, daß man das Reaktionsgas — gegebenenfalls im Gleichstrom mit einem Inertgas — in eine erste Kondensationsstufe, deren Temperaturgefälle zwischen dem Siedepunkt der eingesetzten Methylen- bzw. Methylverbindung und dem Siedepunkt des jeweiligen in der Reaktion gebildeten Nitrils liegt, einführt, fraktioniert kondensiert, das kondensierte rohe Nitril als Sumpfprodukt abzieht, den nicht kondensierten Anteil, der in der Hauptsache aus der nicht umgesetzten Methyl- oder Methylenverbindung und Chlorcyan sowie aus dem während der Reaktion gebildeten Chlorwasserstoff und den Nebenprodukten besteht, in eine zweite Kondensationsstufe führt, deren Temperaturgefälle zwischen Zimmertemperatur und dem Siedepunkt der Methyl- oder Methylenverbindung liegt, kondensiert, das dabei anfallende zweite Kondensat aus restlicher Methyl- oder Methylenverbindung abzieht und bevorzugt das hierbei anfallende Abgas aus Chlorwasserstoff und gegebenenfalls Inertgas in eine dritte Kondensationsstufe einbringt, in der man den Chlorwasserstoff und gegebenenfalls das Inertgas bei Temperaturen von +10°C bis −100°C, bevorzugt von −10 bis −70°C, von geringen Resten an organischen Verunreinigungen trennt, worauf man das anfallende rohe Nitril in bekannter Weise destilliert und gegebenenfalls in bekannter Weise weiter reinigt; das in der zweiten Kondensationsstufe anfallende Kondensat bevorzugt in die Reaktionsstufe zur Herstellung von Nitril zurückführt und bevorzugt den Chlorwasserstoff aus dem die dritte Kondensationsstufe verlassenden Gas durch Auswaschen mit Wasser gewinnt.

**Claim**

A process for the production of nitriles which are produced by the reaction of cyanogen chloride with aromatic or heteroaromatic methylene compounds or methyl compounds which contain activated hydrogen, and working up the reaction mixture, characterised in that the reaction gas mixture resulting after the reaction at from 500 to 1200°C is condensed in the successive temperature stages such that the reaction gas, optionally in co-current flow with an inert gas is introduced into, and subjected to fractional condensation in a first condensation stage, the drop in temperature of which lies between the boiling point of the methylene or methyl compound used and the boiling point of the respective nitrile formed in the reaction, the condensed crude nitrile is drawn off as a sump product, the uncondensed proportion which mainly consists of the unreacted methyl or methylene compound and cyanogen chloride and of the hydrogen chloride which is formed during the reaction and the by-products is introduced into, and condensed in a second condensation stage, the drop in temperature of which lies between room temperature and the boiling point of the methyl or methylene compound, the second condensate resulting thereby consisting of residual methyl or methylene compound is drawn off and the waste gas resulting thereby of hydrogen chloride and optionally inert gas is preferably introduced into a third condensation stage, in which the hydrogen chloride and optionally the inert gas is separated from small residues of organic impurities at a temperature of from +10 to −100°C, preferably from −10 to −70°C, whereupon the resulting crude nitrile is distilled in a known manner and optionally further purified in a known manner; the condensate which is produced in the second condensation stage is preferably returned into the reaction stage for the production of nitrile and the hydrogen chloride is preferably obtained from the gas leaving the third condensation stage by washing with water.

**Revendication**

Procédé pour l'obtention de nitriles qui sont préparés par réaction de chlorure de cyanogène avec des composés méthyléniques ou méthyliques, aromatiques ou hétéro-aromatiques, qui contiennent de l'hydrogène activé, et traitement du mélange réactionnel, procédé caractérisé en ce que l'on condense le mélange de gaz de la réaction sortant après cette réaction à 500 à 1200°C, à des paliers successifs de température, que l'on introduit le gaz de la réaction, éventuellement en courant parallèle avec un gaz inerte, dans une première étape de condensation dont le gradient de température se situe entre le point d'ébullition du composé méthylénique ou méthylique mis en oeuvre et le point d'ébullition du nitrile qui s'est formé chaque fois dans la réaction, condense en fractionnant, extrait le nitrile brut condensé comme produit de fond, envoie la partie non-condensée, qui est constituée, pour la majeure partie du composé méthylénique ou méthylique et du chlorure de cyanogène qui n'ont pas réagi, ainsi que de l'acide hydrochlorique formé pendant la réaction et des produits secondaires, dans une seconde étape de condensation dont le gradient de température se situe entre la température ambiante et le point d'ébullition du composé méthylique ou méthylénique, condense, reprend le second condensat obtenu ici, constitué du reste du composé méthylique ou méthylénique, et amène le gaz qui s'échappe ici, constitué par l'acide hydrochlorique et éventuellement le gaz inerte dans une troisième étape de condensation, où l'on sépare l'acide hydrochlorique et éventuellement le gaz inerte, à des températures de +10°C à −100°C, de préférence de −10 à −70°C, des faibles restes des impuretés organiques, après quoi on distille, de la façon connue, le nitrile brutobtenu, et éventuellement poursuit sa purification de la façon connue, le condensat obtenu dans la deuxième étape de condensation étant retourné de préférence dans l'étape de réaction pour la préparation de nitrile, et récupère l'acide chlorhydrique par lavage avec de l'eau du gaz de la troisième étape de condensation.

Abb.1

0 034 763